# EUROPEAN PATENT SPECIFICATION

(11) **EP 4 103 044 B1**
(45) Date of publication and mention of the grant of the patent: **21.08.2024**
(21) Application number: 21710568.3
(22) Date of filing: 12.02.2021
(51) Int. Cl.: A61B 5/00, A61B 5/03, A61B 1/31

(54) **DEVICE FOR DETECTING PROSTATE FEATURES**
VORRICHTUNG ZUM NACHWEIS VON PROSTATAMERKMALEN
DISPOSITIF DE DÉTECTION DE CARACTÉRISTIQUES DE LA PROSTATE

(30) Priority: 13.02.2020 IT 202000002914
(43) Date of publication of application: 21.12.2022
(73) Proprietor: VI Biotech S.r.l., 36100 Vicenza (IT)
(72) Inventor: TROILO, Angelica, 36100 Vicenza (IT)
(74) Representative: Braidotti, Andrea
(86) International application number: PCT/IB2021/051164
(87) International publication number: WO 2021/161233

(56) References cited:
- EP-A1- 2 322 110
- US-A- 5 836 894
- US-A- 6 142 959
- US-A1- 2007 293 792
- US-A1- 2009 093 733
- US-A1- 2010 145 379

## Description

The present invention relates to a device for detecting the characteristics of the prostate, in particular for the purpose of identifying potentially pathological conditions of the prostate itself.

It is known that prostate cancer is one of the most common cancers in the male population and represents about 15% of all cancers diagnosed in men: the estimates, for the year 2017, speak of 34,800 new cases a year in Italy. Although the risk of the disease having a fatal outcome is low, in order to further decrease this risk it is important to intervene in time.

General population screening programs have been and remain the subject of debate at various levels: health, economic, social. In terms of the health service offered by the institutions, the perplexities concern the risks of overdiagnosis, complications from biopsy, unnecessary interventions, psychological distress, unnecessary commitment of the diagnosis and treatment structures.

However, considering the mortality reduction data, it is appropriate that men, in particular between the ages of 50 and 75, in health conditions that allow them to undergo radical therapies and with a life expectancy of more than 10- 15 years old, are informed of the possibility of a diagnostic approach for the early recognition of prostate cancer, as well as of the risks and benefits that this diagnostic approach entails.

In order to contain the drawbacks indicated above, personalized screening programs have been proposed more recently on the basis of individual risk factors such as ethnicity (greater risk for African-American patients) and family history. The presence of more subjects with prostate cancer in first-degree relatives arising at a young age (age <55 years) represents the main element of suspicion for a possible hereditary predisposition, although at the moment there are no specific genetic tests to be recommended to recognize the individuals at risk.

The diagnosis of prostate cancer (pCa) takes place thanks to the agreement of a plurality of diagnostic tests, of which those currently standardized in clinical practice are:
- the analysis of some biochemical markers, primarily the dosage of the Specific Prostatic Antigen (Prostate Specific Antigen - PSA) in serum, i.e. the dose of a specific enzyme present in the blood,
- the physical examination of the patient performed by the doctor through digital exploration of the prostate (Digital Rectal Examination - DRE),
- some specific radiological tests: multiparametric prostatic magnetic resonance imaging, PET-CT with choline or PSMA (the latter test used mainly for post-diagnosis clinical staging).

However, PSA testing is not fully satisfactory as this enzyme is a protease produced by both normal and malignant prostate epithelial cells. Therefore, this test is specific for the prostate, but not for prostate cancer, as the PSA can be altered, as well as in patients with prostate cancer, also in patients with prostatitis, benign hyperplasia and even after a biopsy, giving therefore life to cases of false positives. It should also be remembered that with the commonly used PSA threshold values (4 ng/ml) false negative results can be found in 20-25% of the initial clinically significant tumors; and that the most undifferentiated tumors may begin with within-threshold PSA values. Therefore, in addition to the risk of a false alarm, the risk of a "false reassurance" given by a negative test result must also be taken into consideration.

The DRE is not fully satisfactory as it is subject to the sensitivity of the operator who performs it and does not detect non-palpable tumors. This results in the procedure having inherently low sensitivity levels.

More accurate confirmation can be obtained by combining the two investigation techniques, which however require diagnostic confirmation to be obtained with another technique. This can consist of a histological examination obtained from a prostate biopsy, which can be performed with a transrectal or transperineal technique, where the cutting needle - which retrieves the histological sample - is guided by a transrectal ultrasound (TRUS).

However, due to the complexity of the biopsy examination, the need to perform it must be based not on a single detection of PSA alteration, but on a plurality of concordant tests, such as for example an alteration of PSA and/or suspicion on rectal exploration and/or suspicious imaging.

Commonly used imaging techniques are for example
- transrectal ultrasound, which is indicated above all as a guide to biopsy,
- multiparametric magnetic resonance imaging (MRI) which is proposed as the best method to define the local extension of the tumor (including locoregional lymph nodes). The MR mp exam involves reading the images obtained with the three sequences according to the so-called PI-RADS methodology (Prostate Imaging Reporting and Data System, or Reporting of Prostatic Images and Related Data) which allows to obtain a "final score" lesions expressing the risk of malignancy of the lesion (PI-RADS 1 = very low risk; PI-RADS 2 = low risk; PI-RADS 3 = intermediate risk; PI-RADS 4 = high risk; PI-RADS 5 = very high risk). Consequently, the MRI examination represents an excellent method to support and guide subsequent choices (prostate biopsies possibly targeted on suspicious areas).

Currently, MRI of the prostate is indicated in subjects who have already performed a first biopsy set with a negative result, before undergoing a second biopsy, in case the suspicion of neoplasm persists; and, again, it can be used in subjects who have been placed under active surveillance, before the biopsy check required by the protocols. Furthermore, in the staging phase, it could be used before a radical prostatectomy to plan the type of surgery itself.

However, the present methods are not fully satisfactory as they are highly invasive, and require the use of complex instruments and qualified personnel, thus making access to screening complex for a large portion of the population.

EP2322110 discloses a device for measuring defecation reflexes which is provided with means for electrical neurostimulation. In particular, this solution comprises a probe with a tubular section for insertion and with an expandable balloon which can assume a first condition (of deflation) in which it is housed inside the tubular section and a second condition (of inflation) in which it comes out. radially beyond the inserted end of the tubular section. Furthermore, the probe comprises pressure sensors mounted on the tubular section and also a further pressure sensor to measure the pressure inside the balloon, and thus define a measure of the pressure exerted by the rectal walls on the balloon following the neurostimulation signals generated by electrodes mounted on the tubular section of the probe.

US6142959 describes a device for the graphic representation of the prostatewhich comprises a probe to be inserted inside the rectum and which is provided at the end of a head portion with a group of pressure sensors; moreover, the head portion is then associated with a handle by means of a section shaped like a rod and an external disk is also provided to have the rotation references of the probe with respect to its own axis.

US2010/145379 discloses a rectal balloon apparatus for immobilizing the region around the prostate. This apparatus includes a stem having a liquid passage extending therethrough; additionally, a rectal gas delivery lumen can be associated with the rod, and/or integrated into the rod, for thheremoval of rectal gas or other fluids.

US5836894 describes a device for detecting geometric and mechanical parameters through which to make a representation of the prostate; this device is equipped with pressure sensors to measure the stresses on the soft tissues. In particular, the device comprises a transrectal probe with a stem, a position sensor for detecting the position of the tip of the stem and with a matrix of pressure sensors positioned on the tip of the stem.

US2009/0093733 discloses a device for measuring the transrectal prostate temperature. This device comprises a grip handle, a stem extending from the handle and a probe positioned at the end of the stem and equipped with a temperature sensor.

US2007/0293792 discloses an apparatus which comprises a force or pressure sensor and/or a temperature sensor which is mounted on a probe that can be inserted through the rectum.

The object of the invention is to propose a device for detecting the characteristics of the prostate, preferably in order to identify potentially pathological conditions of the prostate itself, which allows to overcome - at least in part - the drawbacks of traditional solutions.

Another object of the invention is to propose a device that can also be used in total autonomy by the user, without having to resort to a doctor.

Another object of the invention is to propose a device which is simple, quick, intuitive and repeatable to use.

Another object of the invention is to propose a device which allows to detect changes in the consistency of the prostate tissues.

Another object of the invention is to propose a device which allows to detect dimensional/volumetric and/or morphological alterations of the prostate tissues.

Another object of the invention is to propose a device which is alternative and improved with respect to known solutions.

Another object of the invention is to propose a device which does not cause allergies or injuries in general.

Another object of the invention is to propose a device which is simple to maintain or which does not require any maintenance.

Another object of the invention is to propose a device which is safe for the user.

Another object of the invention is to propose a device that can be used by a large number of people.

Another object of the invention is to propose a device which can be manufactured simply, quickly and with low costs.

All these purposes, either alone or in any combination thereof, and others which will result from the following description, are achieved, according to the invention, with a device for detecting the characteristics of the prostate, in particular for the purpose of identifying potentially pathological conditions of the prostate itself, as defined in claim 1.

The present invention is further clarified hereinafter in a preferred embodiment thereof reported for purely illustrative and non-limiting purposes with reference to the attached drawing tables, in which:
- Figure 1: shows in schematic side section of the device according to the invention in inactive configuration,
- Figure 2: shows it in the same view as fig. 1 but in the insertion configuration and ready for its activation,
- Figure 3: shows it in the same view and configuration of fig. 2 but in a different state,
- Figure 4: shows it in schematic side section with the expandable element in the activated condition,
- Figure 5: shows it in perspective view, without the handle, and with the expandable element in the same condition as fig. 4,
- Figure 6: shows an enlarged schematic transverse section of it, and
- Figure 7: shows another construction detail in section.

As can be seen from the figures, the device 1 according to the invention comprises an introducer element 2 (hereinafter simply "introducer") for stabilizing the device with respect to the anus of a user.

In particular, the introducer 2 is configured to be inserted - at least partially - inside the user' s anus and to be stabilized in the working position.

Conveniently, the introducer 2 comprises an internally hollow shaped body and, preferably, it is an externally shaped tubular body. Conveniently, the introducer 2 has a configuration - both in terms of shape and size - so as to be anatomically hooked and stabilized to the user' s anus.

Preferably, for this purpose, the introducer 2 has a substantially circular cross section and comprises a swollen upper part 4 with a maximum diameter advantageously of about 15-18 mm, a flattened lower part 6 with a diameter advantageously of about 50 mm and with a intermediate neck 8 for connection.

Conveniently, the swollen upper part 4 of the introducer 2 is configured to be inserted inside the anus of the user, and therefore has a shape substantially tapered towards the end and rounded in the underlying part, so as to penetrate more easily but without the risk of causing injury to the user.

Conveniently, the lower part 6 of the introducer 2 is configured to rest externally on the perineal area of the user, and for this purpose the surface of the lower part 6 of the introducer 2 facing the upper part 4 of the same preferably has a certain concavity of connection with the neck 8 of the introducer itself, followed towards the external edge by a slight convexity.

Conveniently, the neck 8 of the introducer 2 is configured to be positioned and retained by the anal sphincter of the user, and for this purpose has a reduced section and such as to be minimally invasive during use.

The introducer 2 presents a through axial cavity 10, which - suitably - involves its upper part 4, its neck 8 and its lower part 6. In particular, the through axial cavity 10 has a diameter suitable for the passage of a insertion element 12. Preferably, the insertion element 12 is finger-shaped. Alternatively, in a variant not shown here, the insertion element 12 can have a different configuration, for example substantially egg-shaped or with radial swellings along its longitudinal development.

Conveniently, the insertion element 12 is an element which is movable with respect to the introducer 2.

Conveniently, the introducer 2 internally comprises an axial through cavity 10 within which the insertion element 12 slides. Preferably, the cross section of said through axial cavity 10 is closed, i.e. it is not radially connected with the outside. Conveniently, the introducer 2 comprises entirely or in at least a part of it an external "saddle" conformation such as to allow its insertion inside the anus of the user. Preferably, for this purpose, the introducer 2 comprises a conformation which is substantially defined by two truncated cones joined together at their respective minor bases and crossed internally by an axial through cavity 10 within which the insertion element 12 slides.

Advantageously, the introducer element 2 defines/comprises a crown for positioning and stable fixing of the device 1 on the user' s anus. The insertion member 12 is a member configured to be introduced/inserted through the anal sphincter and anus into the user' s rectum. Preferably, for this purpose, the insertion element 12 has a substantially cylindrical and elongated shape; moreover, preferably, the insertion element 12 has the upper end 14,i.e.the end intended to be introduced into the user' s rectum, which is rounded. The upper end 14 of the insertion element 12 also has a diameter slightly greater than the diameter of the remaining part of the insertion element 12 and, in particular, of the axial through cavity 10, so as to hinder its complete extraction from the introducer 2.

Advantageously, the shape of the upper end 14 of insertion element 12 and the shape of the upper part 4 of the introducer 2 are such that in the inactive configuration of the device 1 - that is, when the insertion element 12 is located with respect to the introducer 2 so that the upper end 14 of the insertion element is in contact and/or close to the upper part 4 of the introducer 2 - they present/define a substantial continuity of the external profile of the assembly defined by them, allowing thus a simple and practically painless introduction of the insertion element 12 of the device 1 into the user' s anus.

Preferably, the device can comprise a condom (not shown) for introducing the insertion element 12 into the user' s anus; suitably, for this purpose, the insertion element 12 of the device 1 is inserted inside a condom during the introduction of said insertion element 12 into the anus and, once the measurement has been carried out (as will be seen more clear below), the insertion element 12 is extracted from the anus and the condom is withdrawn from said insertion element.

Conveniently, device 1 is configured to assume:
- a first configuration, corresponding to an inactive and/or disconnection configuration of said device, in which the introducer 2 is in contact with or is near the end 14 of said insertion element 12 which is intended to be inserted inside of the user' s rectum,
- a second configuration, corresponding to an insertion configuration of said device, in which the introducer 2 is further spaced from the end 14 of said insertion element 12 which is intended to be inserted inside the user' s rectum.

Conveniently, moreover, the passage from said first configuration to said second configuration, and vice versa, occurs by making the insertion element 12 slide with respect to said introducer 2.

Conveniently, when the introducer element 2 is hooked/positioned on the anus, the latter is substantially spaced apart from the insertion element 12 which slides inside the introducer element 2. Therefore, advantageously, this allows the sliding and introduction of the insertion element 12 into the user's rectum without this coming into contact with the internal walls of the anus, thus allowing a more comfortable and less traumatic introduction. In other words, thanks to the introducer element 2, the contacts of the anus with the insertion element 12 which is sliding are avoided or at least minimized.

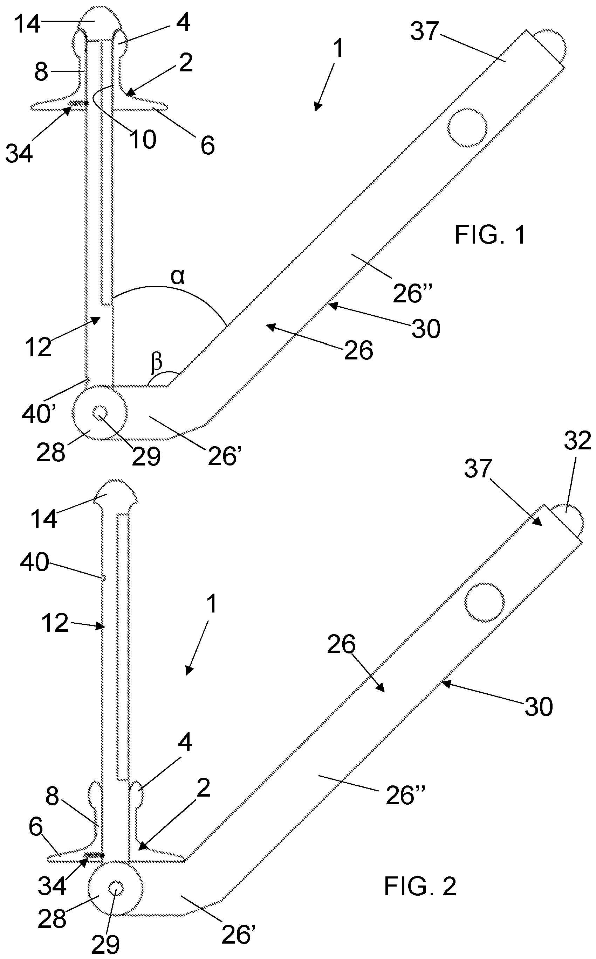

Conveniently, moreover, the stable anchoring/positioning of the introducer element 2 in the anus allows defining a certain and precise spatial reference with respect to the anus of the user himself, so that the sliding of the insertion element 12 with respect to the introducer element 2 it also allows to define/know what said insertion element has been inserted into the anus. Advantageously, by controlling or detecting at the level of tactile feedback (for example with a snap engagement) or visual (for example with suitable marks positioned on the insertion element) the sliding of the insertion element 12 with respect to the introducer element 2, it is possible to define what said insertion element was inserted into the anus. Conveniently, for example, when the device is in the second end-of-stroke configuration (see fig. 2),i.e.when the insertion element 12 has been made to slide with respect to the introducer 2 until it reaches an end-of-stroke position, it is thus certain of the length of the part of the insertion element 12 which appears to have been inserted into the anus.

Conveniently, the device 1 comprises a measuring apparatus - indicated as a whole with the reference "16" - which is associated with the insertion element 12. Conveniently, the measuring apparatus 16 comprises an expandable element 18 which acts as a support and transport for a pressure sensor 20. In particular, the pressure sensor 20 is mounted on the expandable element 18.

Conveniently, the pressure sensor 20 is mounted on the expandable element 18 so that the latter brings said sensor into direct contact with the prostate. Conveniently, the expandable element 18 has no measuring function, but only serves to bring the pressure sensor 20 into direct contact with the prostate.

Preferably, the expandable element 18 is mounted on the insertion element 12.

The pressure sensor 20 is associated with the expandable element 18 and is configured to detect the elasticity of the user' s prostate tissues and/or at least one morphological characteristic and/or size of the user' s prostate. Preferably, the pressure sensor 20 is mounted externally on the expandable element 18 so that, when the latter is in the expanded condition, said sensor comes into direct contact with the prostate. Preferably, the pressure sensor 20 is not located inside the expandable element 18 and does not measure the pressure difference deriving from the deformation of the expandable element 18.

Conveniently, said pressure sensor 20 comprises a mechanical transducer, preferably of the type which transforms a mechanical input action/quantity (in terms of force or pressure) into a variation of color or shade/intensity of color. Preferably, said pressure sensor 20 does not provide for the use of transducers of the type which, at their output, have an electrical quantity/signal. In particular, the pressure sensor 20 does not provide for any electromagnetic detection and/or does not use optical-electric transducers and/or optical fiber sensors. Preferably, said pressure sensor 20 comprises a transducer which outputs a direct measurement, without the need for further processing or means for representing the measurement.

Conveniently, as said, said support element 18 is expandable and can assume a first retracted/contracted condition, of reduced bulk, preferably suitable for allowing said element to be housed in the insertion element 12, and can also assume a second expanded condition, of greater bulk, such as to bring the pressure sensor 20 in correspondence with and/or in contact with the user' s prostate. Advantageously, the insertion element 12 is internally hollow and in its internal cavity 21 is contained in said first contracted condition said expandable element 18 and the pressure sensor 20.

Preferably, the device 1 is configured - from the mechanical and/or mechanical point of view. or electronic and/or pneumatic (for example allowing the fluidic connection of the inflation means with the expandable element) - so that said expandable element 18 can inflate (i.e. it passes from the first retracted/contracted condition to the second expanded condition) only when the device is in said second configuration and, in particular, after the sliding of the insertion element 12 with respect to the introducer element 2 has brought the insertion element into an end-of-stroke position which, conveniently, corresponds to a condition of maximum distance of the end 14 of the insertion element 12 with respect to the introducer element 2.

Preferably, the device 1 is configured - from the mechanical and/or electronic and/or pneumatic point of view - so that its passage from the second to the first configuration (i.e. the sliding of the insertion element 12 so as to bring its end 14 into contact or in proximity to the introducer element 2) can be carried out only when said expandable element 18 is in said first retracted/contracted condition.

Conveniently, said expandable element 18 comprises an inflatable bag 18', preferably of flexible material. Conveniently, the inflatable bag 18' is not elastic or in any case with limited elasticity so as not to undergo substantial variations in surface area when inflated.

Advantageously, in the deflated condition (corresponding to said first retracted/contracted condition of the expandable element 18), the bag 18' is rolled up on itself and is contained in the cavity 21 of the insertion element 12 and, preferably, so as to be facing an opening 23 of the insertion element 12 which runs along the entire length of the bag itself. Preferably, said opening 23 is defined along the lateral surface of the insertion element 12.

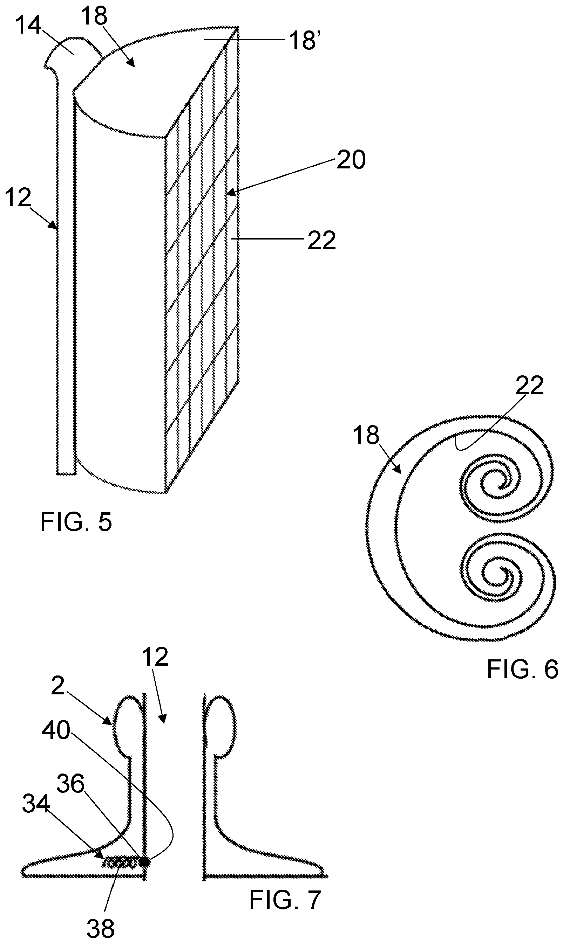

Advantageously, in the inflated condition (corresponding to said first expanded condition of the expandable element 18), the bag 18' can take various shapes, preferably linked to the shape its flat development; in the example shown in fig. 5, the bag 18' substantially has the shape of a semicylinder, that is a cylinder with semicircular bases instead of circular ones, but it could also have a truncated pyramidal shape with a square or polygonal base in general.

Advantageously, in order to make the inflatable bag 18' automatically assume the rolled configuration in the absence of external stresses, i.e. in the absence of pressurized fluid inside it, traditional elements are associated with the walls of the envelope that forms said bag 18', of shape memory material (e.g. Nitinol), which can be applied for example at the upper and lower corners of the bag 18' and which for example can have the shape shown in fig. 6.

Conveniently, the bag 18' is constrained to the insertion element 12 so as to be housed in the cavity 21 of the insertion element itself when it is in a contracted and rolled condition and to protrude partially or entirely through the opening 23 of the latter. cavity, remaining bound to it for example with a thin vertical band thereof, when it is in an inflated condition.

Conveniently, in the inflated condition, the bag 18' has such a conformation as to externally embrace the largest possible surface of the prostate. Advantageously, in the inflated condition, the bag 18' has a shaped surface - preferably substantially C-shaped - which is further away from the insertion element 12.

Advantageously, the measuring apparatus 16 comprises at least one pressure sensor 20 In particular, the pressure sensor 20 is associated with the - preferably mounted on the - inflatable bag 18' so that, in the inflated condition of said bag, the pressure sensor 20 is positioned in correspondence with the user' s prostate and, suitably, is in direct contact with the latter.

Conveniently, the pressure sensor 20 is applied to the external surface/area of the bag 18' which is intended to be brought close to and/or in contact with the user's prostate. Preferably, for this purpose, the pressure sensor 20 is applied on a surface of the bag 18' which is suitably shaped to embrace/wrap, at least partially, the user' s prostate.

Advantageously, the pressure sensor 20 comprises a film 22 (or in any case a substantially laminar developing element) sensitive to pressure (i.e. it is a pressure sensitive film), preferably made with a material that changes color and/or shade/intensity of color based on the pressure exerted externally on it. For example, this material can advantageously be constituted by metallic nanoparticles whose organization can be modified during the application of a pressure, leading to a variation of the plasmonic resonances which generate their color.

Alternatively, the film 22 can be composed of a multilayer, in which a layer is made up of material containing chromophores, and a further layer is configured to allow said chromophores to imprint their own color, as for example in the case of the Prescale product. ^{®}, or polymers (or polymer networks) that react to deformations by modifying their absorption spectra in the visible.

Conveniently, the pressure sensor 20 can change color or shade/intensity of color when subjected to a compression and more specifically it can exhibit a certain color or shade/intensity when subjected to a minimum threshold pressure, and it can vary abruptly or in progressive/gradual way of color or gradation/intensity when subjected to higher pressure. More specifically, the color or the gradation/intensity can change progressively as the pressure increases up to, for example, 90KPa.

Conveniently, the pressure sensor 20 records/detects the pressure exerted externally on the film 22 mounted on the inflatable bag 18' and which derives from the contact of said film with the tissues of the prostate, and therefore from the resistance and force opposed by said tissues to the inflation force of the bag 18' on which the film 22 is mounted.

Conveniently, the film 22 is configured in such a way that, when subjected to a certain compression, it changes color or shade/intensity of color permanently (i.e. unchangeable over time); moreover, once the color or shade/intensity of color has changed, this new color or new shade/intensity of color remains substantially stable.

Conveniently, for example, the film 22 can have a sensitive surface of about 10 cm in height and 5 cm in width, so as to be able to face, in conditions of use, at least a portion thereof in correspondence with and/or in proximity to the prostate of the user.

Advantageously, the film 22 can comprise a grid or net conformation/arrangement, so as to help the localization of the points which have undergone greater pressures.

Advantageously, the film 22 can be made in a single body or by several elements mutually connected so as to be movable with each other. Advantageously, the elements of the film 22 are intertwined with each other so as to form meshes; preferably, moreover, in correspondence with the junction nodes, said elements are mobile (in translation and/or in rotation) along at least one axis, preferably along two or three axes (defining for example a Cartesian triple X, Y and Z) substantially perpendicular to each other.

Advantageously, the pressure sensor 20 can comprise a matrix of pressure transducers.

Conveniently, the expandable element 18 is connected to means, preferably inflating means, configured to cause its passage from said first retracted condition to said second expanded condition. In particular, said inflation means are configured to fill the expandable element 18 with a fluid (liquid or gas) so as to cause its expansion/dilation, thus increasing its volume, thus bringing the pressure sensor 20 into contact. with prostate tissues.

Correspondingly, the expandable element 18 is connected to means, preferably deflation means, configured to cause its passage from said second expanded condition to said first retracted condition. In particular, said deflation means are configured to release the fluid (liquid or gas) from the expandable element 18 so as to thus decrease its volume and cause it to contract, thus removing the pressure sensor 20 from the prostate tissues and bring it back towards the insertion element 12. Preferably, the expandable element 18 retracts returning to assume - thanks to the presence of shape memory material elements - said first retracted condition, thus allowing its re-entry into the cavity 21 of said insertion element.

Advantageously, for this purpose, the bag 18' (which defines said expandable element 18) is connected by means of one or more tubes to a tank (not shown), which can belong to the device 1 or can be external to it and which is aimed at containing a fluid, preferably a liquid, intended to be introduced by pressure into the bag 18' to inflate it, thus making it pass to the inflated condition, and also intended to escape and/or to be extracted from said bag 18 to deflate it, thus making it return to the deflated condition.

Conveniently, the passage of the fluid through the tube in the direction of the bag 18' and/or in the opposite direction can be caused by a pneumatic system comprising a pump or a syringe positioned externally or internally to the device 1. Advantageously, the passage of the fluid through the the small tube in the direction of the bag 18' and/or in the opposite direction can be controlled by at least one suitable regulating valve.

In the event that the passage of the fluid is caused by a pneumatic system, a control unit is advantageously provided which is operated by a suitable actuator, controlled for example by a button 27, and which controls the operation of the pump and/or which it controls the opening/closing of a valve, and therefore the inflation and deflation of the bag 18',

When the bag 18', which is initially inactive and is contained in a wound/retracted condition inside the insertion element 12, is filled with the fluid, swells, and therefore emerges from the cavity 21 through the opening 23 of the insertion element 12.

Conveniently, the dimensions and arrangement of the various parts are defined so that in the inflated condition of the bag 18' inside the user' s rectum, the film 22 applied to the bag 18' faces and in proximity and/or contact with the user' s prostate.

Conveniently, the introducer 2 and the insertion element 12 are equipped with locking means 34 cooperating with each other and able to make the insertion element assume at least a predefined stable position, preferably elastically stable, with respect to the introducer 2.

Conveniently, the locking means 34 are configured to define at least one stable position of the insertion element 12 with respect to the introducer 2, in particular so as to prevent movement (sliding) of the insertion element 12 with respect to the introducer 2.

Preferably, the locking means 34 are configured to define an elastically stable position of the insertion element 12 with respect to the introducer 2 when said device 1 is in the insertion configuration (see fig. 2), i.e. when the insertion element 12 it is at least partially inserted into the user' s rectum; suitably, in this configuration, the introducer 2 is spaced with respect to the upper end 14 of the insertion element 12 and, preferably, is in correspondence with the lower end 28 of said insertion element.

Preferably, the locking means 34 are configured to define a further elastically stable position of the insertion element 12 with respect to the introducer 2 when said device 1 is in an inactive or disconnected configuration (see Fig. 1), i.e. when the insertion element 12 is disconnected with respect to the user' s rectum; suitably, in this configuration, the introducer 2 is in contact and/or close to the upper end 14 of the insertion element 12.

For example, these locking means 34 can comprise a protruding element 36 (for example a pin or a sphere) positioned at least partially inside the cavity 10 of the introducer 2 and coupled to a spring 38 (or other elastic means) which can push said protruding element 36 to engage corresponding recesses 40,40' arranged in correspondence with these predefined positions, which the insertion element 12 must be able to assume with respect to the introducer 2.

Conveniently, means are also provided that can be controlled by the user (or by an operator) for activating and deactivating said locking means 34.

Means are also provided for disengaging the element 36 protruding from the corresponding recesses 40,40', in which it is alternately engaged and these means (not shown) can be advantageously body consisting of a button or an actuator in itself traditional. Moreover, it is also provided that the recesses 40,40' are configured in such a way as to be able to remove the protruding element 36 with a suitable pressure exerted on the same finger insertion element 12.

Preferably, a first recess 40 can be positioned so that when this is engaged by the protruding element 36, the device 1 is in said inactive or disconnected configuration (see Fig. 1), i.e. its upper end 14 is substantially in contact with the upper part 4 of the introducer 2; and a second recess 40' can be positioned so that, when this is engaged by the protruding element 36, the device 1 is in the insertion configuration, i.e. the upper end 14 of the insertion element 12 is further away from the upper part 4 of the introducer 2.

The device 1 according to the invention comprises an extractor means 30 which is intended to always remain, at least in part, external to the user' s anus, to thus define a portion of the grip by the user, or of another subject, in order to extract the insertion element 12 from the anus of the user.

Conveniently, the extractor means 30 can also be used to define a gripping portion by the user, or by another subject, in order to make the insertion element 12 slide with respect to the introducer element 2 so as to introduce the insertion element 12 into the user' s rectum.

Conveniently, said extractor means 30 can also be configured to cause the insertion element 12 to slide with respect to the introducer element 2, to thus make the device pass from said first configuration to said second configuration and/or vice versa.

Conveniently, said extractor means 30 can comprise a handle 26 or, in a variant not shown here, a cable or other wire extraction element which is associated with the insertion element 12.

Preferably, the extractor means 30 comprises a handle 26 which is associated with the insertion element 12 and always remains (i.e. in any configuration of the device) external with respect to the rectum and the anus of the user. In particular, at least a part of said handle 26 defines a handle intended to be grasped, gripped and held in the hand (i.e. gripped) by the user, or by another person.

Preferably, the handle 26 is configured to facilitate the user in pushing the insertion element 12 through the introducer 4, to thus pass the device 1 from the inactive configuration to the insertion one.

In an embodiment not shown, the handle 26 can be an extension of the insertion element 12 and be aligned with the latter; suitably, the handle 26 and the insertion element 12 can be defined by distinct portions of a single piece.

Advantageously, in the embodiment shown, the handle 26 is defined by a piece distinct from the insertion element 12 and articulated to the latter, in particular by means of a hinge 29. Preferably, the lower end 28 (i.e. the end which - in the configuration introduced of the device 1 - it is external, or in any case more spaced, with respect to the user' s rectum) is articulated to a terminal portion of the handle 26 which is opposite with respect to the other terminal portion 37 which, suitably, is the one intended for be challenged.

The hinge 29, which articulates the handle 26 with the insertion element 12, allows to adjust the angle α formed between them, so as to exert a progressive and precise thrust on the insertion element 12 (which thus causes the sliding of the insertion element 12 with respect to the introducer 2, and thus makes the device pass from the inactive/disconnection configuration to the insertion one), maintaining a comfortable position, especially in case of use of the device 1 by the same user. Preferably, the angle α formed by the insertion element 12 with the handle 26 is indicatively comprised between a minimum of about 45° and a maximum of about 180°.

Advantageously, the hinge 29 does not constitute a free joint, but frictioned or blocked, so as to ensure a certain stability of the angle α and to require a certain force for its variation. This in order to prevent an involuntary modification of that angle during the use of the device 1.

Advantageously, in a particular embodiment of the device 1 according to the invention, the hinge 29 can be controlled by an actuator controlled by a button 32, preferably of the "vigilant" type (i.e. of the type that allows the clutch or lock to be released only when actuated, and keeps it locked when not actuated). Alternatively, this button 32 can be of the type which, when pressed, blocks the reciprocal rotation of the insertion element 12 with respect to the handle 26. The button 32 can be advantageously positioned at the end portion 37 of the handle 26 opposite to that of the handle, hinging to the insertion element 12.

The button 32 which controls the hinge 29 can be advantageously coordinated in its interventions with the button 27 which controls the inflation and deflation of the bag 18', In particular, the button 27 can be activated to cause the filling and/or emptying of the bag 18' only when the button 32 is deactivated and therefore the articulation of the insertion element 12 to the handle 26 is locked.

Preferably, the button 27 can be arranged in correspondence with the position occupied by the index insertion element of the hand of the user (or of another person) which holds the handle 26 while using the device 1, while the button 32 can be positioned substantially in correspondence with the position occupied by the thumb insertion element of the user' s hand which grips the handle 26 while using the device itself.

Conveniently, the handle 26 can be substantially divided into two portions 26', 26" which are angled to each other and, in particular, form an angle β between them, preferably of about 135°. Conveniently, the device 1 is configured so that, in said second configuration (i.e. when the insertion element 12 is inserted in the rectum), the portion 26' (i.e. the one closest to the hinge 29) of the handle 26 abuts with the introducer 2, thus substantially defining a limit switch for the sliding of said insertion element 12 with respect to said introducer 2.

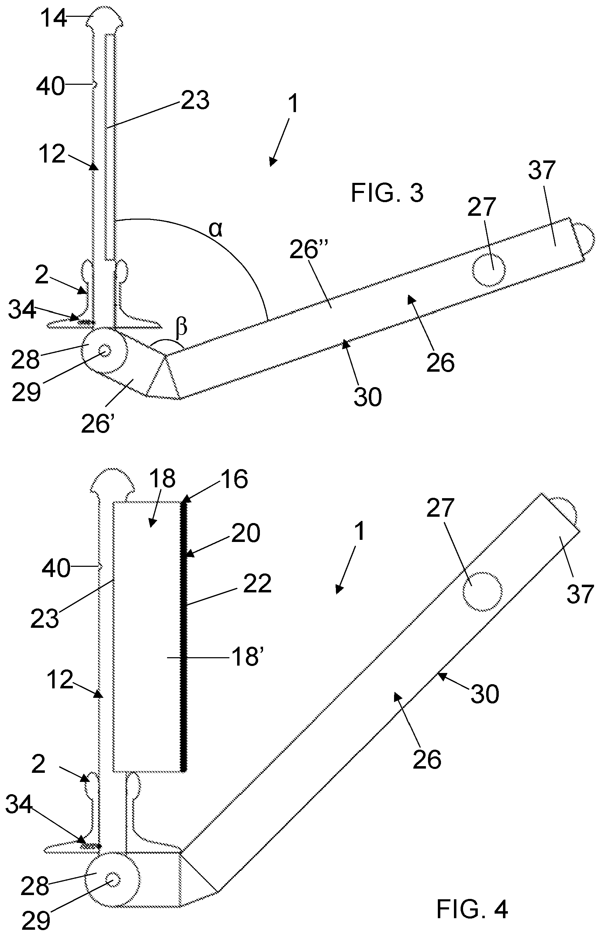

The first portion 26', closest to the hinge 29, can preferably have a dimension linked to that of the lower end of the introducer 2, so that when the insertion element 12 is in a position ready for activation (fig. 2) or already activated (fig. 4), the portion 26' adheres to the lower surface of the introducer 2.

Suitably, it is also envisaged that the device 1 according to the invention comprises a control unit, which oversees the operation of the device during its use and which is also advantageously configured to detect (through suitable sensors) the position of the insertion element 12 with respect to the introducer 2 and/or the angle α formed between the insertion element 12 and the handle 26. Preferably, this control unit comprises a processor, for example a microcontroller, which is suitably configured to control - and preferably coordinate - the inflation and/or deflation of the expandable element 18 and/or the activation/deactivation of the locking means 34 and/or the joint (through the hinge 29), and hence the angle, between the insertion element 12 and the handle 26.

Conveniently, all the components of the device 1 can be made of biocompatible and/or hypoallergenic materials, such as stainless steel, aluminum or polymeric materials. Preferably, the materials are non-porous so as to simplify their use and reduce the difficulty in maintaining them.

Furthermore, the material with which the expandable element is made, and in particular the bag 18', is substantially impermeable and preferably consists of a material and/or a set of materials that are not permeable to the filling fluid used for its inflation, so as to avoid spilling of said fluid inside the rectal cavity of the user.

The use of the device 1 according to the invention clearly results from what has been said above.

When the device 1 is in an inactive or disconnected configuration (see fig. 1), i.e. with the upper end 14 of the insertion element 12 blocked - by means of the locking means 34 - in correspondence (in contact and/or in close position) with the introducer 2, the insertion element 12 is inserted inside the anal orifice until the sphincter is arranged around the neck 8 of the introducer 2, and its lower portion adheres stably to the perineal wall of the user himself.

Subsequently, the locking means 34 are deactivated so that the insertion element 12 can slide with respect to the introducer 2 and can be pushed inside the rectum following a suitable maneuver performed with the handle 26, thus making the device pass from the inactive configuration (see fig. 1) to the activation configuration (see fig. 2).

Conveniently, in the configuration of insertion of the device 1 (i.e. when the insertion element 12 is inserted inside the rectum), the introducer 2 is spaced apart from the upper end 14 of the insertion element 12 and, in particular, it is in contact with and/or in the vicinity of the lower end 28 of said insertion element.

If necessary, this maneuver can be carried out with the control of the button 32 so as to be able to adjust the angle α formed by the insertion element 12 with the handle 26, and thus make the maneuver more comfortable and without effort on the part of the operator.

Subsequently, the first portion 26' of the handle 26 is preferably brought to adhere to the lower surface of the introducer 2, so that the angle formed by the second portion of the handle 26" with the insertion element 12 is substantially equal to approximately 45°, and in this configuration the insertion element 12 is locked by means of the locking means 34.

Conveniently, this stabilization between the various parts allows to obtain a measurement which has a reference system fixed in space and which is therefore repeatable. More in detail, the assembly which includes:
- the introducer 2 which is anatomically attached to the anus in order to define a first spatial constraint,
- the insertion element 12 which, once the hinge 29 is locked, is stabilized with respect to the handle 26 so as to define a second spatial constraint; suitably, once well grasped and held still by the user, the handle 26 - depending on the position chosen by the user (supine, sideways, for example on a bed or chair) - forms with the insertion element 12 the minimum angle,
allows to define an absolute and immobile spatial reference with respect to the prostate and this, suitably, is a condition that allows for a reaction of the prostate tissues once said tissues come into contact with the film 22 - or are compressed by the latter - which is pushed by the expanding bag 18',

Subsequently, therefore, the first button 27 is actuated, which through the control unit commands the introduction of a fluid towards the bag 18', In this way the bag 18' swells, emerges from the cavity 21 of the insertion element 12 through the opening 23, expands inside the rectum of the user until its surface, to which the film 22 is applied, adheres to the user' s prostate.

Subsequently, the control unit controls the pump and/or a suitable valve so as to regulate the flow of fluid through measurements of its internal pressure, until a preset limit pressure is reached, for example 90KPa, or until the maximum volume of the bag 18' is not reached.

Conveniently, in substance, the inflation of the bag 18' causes the compression of the prostate tissues and, through the pressure sensor 20 mounted on said bag 18', the consistency of the prostate tissues - i.e. their elastic stiffness/elasticity - is measured. during and/or following said compression. Conveniently, depending on the elastic stiffness/yielding of the prostate tissues, a normal condition (corresponding to that of a healthy subject), a dubious condition (connected for example to prostatic hypertrophy) or a risk condition (for example, example potentially linked to a neoplasm) which necessarily requires further investigation. Preferably, for this purpose, the film 22 of the pressure sensor 20 is configured to assume three different colors (for example, green/yellow/red) and/or three different shades/intensities of color, depending on the elastic stiffness/compliance of the prostate tissues corresponding to the above three conditions (i.e. normality/doubt/risk).

In other words, the expansion of the expandable element 18 causes compression of the prostate tissues and, in particular, following the application on the prostate tissues of a force substantially perpendicular or variously angled with respect to said, the pressure sensor 20 mounted on said bag 18' it measures the alteration of the state of elasticity of said fabrics, an alteration which can be locally focused or diffused.

Subsequently, after a predetermined time, or after an appropriate actuation of the button 27 and/or the button 32, the control unit commands the pump and/or a suitable valve so as to cause a flow of the fluid in the opposite direction, to deflate so the bag 18'; suitably, in this way, the bag 18' is rolled up again - preferably automatically against the material of which it is made - to be thus re-housed inside the cavity 21 of the insertion element 12.

The locking means 34, which allow the insertion element 12 to slide relative to the introducer 2 until the inactive configuration of the device 1 is reached (see Fig. 1).

Finally, after the locking means 34 have been operated again to lock the insertion element 12 in this inactive configuration of the device, the latter (and in particular the introducer 2) can be completely extracted from the user' s anus.

Conveniently, once the device 1 has been extracted from the user' s anus, the readings made by the pressure sensor 20 can be checked and analyzed, in particular by making the bag 18' come out of the insertion element 12 to thus observe the film 22.

Conveniently, as mentioned, the pressure-sensitive film 22 records on its surface the reaction of the prostate tissues to the thrust force of inflation of the bag 18', whereby the more elastic physiological tissues yield to the force and do not affect the film, and for example, they do not cause any variation in color or color gradation; the stiffer/harder physiological tissues, potentially pathological, by contrasting greater resistance, affect the film causing a variation in color or color gradation, and preferably a different variation in color/gradation depending on how much the opposing force is greater. Advantageously, therefore, a map of the forces (pressures) opposed by the tissues of the prostate to the inflation thrust of the bag 18' is recorded on the film 22.

Advantageously, the map - thus obtained - recorded on the film 22 then allows to detect the alterations in the consistency of the prostate, in particular in terms of elastic stiffness/compliance, and/or the morphological and/or dimensional characteristics of such alterations.

Conveniently, the device 1 does not comprise any means for electrical stimulation of the tissues.

As it clearly appears from what has been said, the device 1 according to the invention is advantageous, indeed optimal, since:
- it allows the user to carry out a prostate exam in a simple, rapid, autonomous and repeatable way; in particular, since it can be carried out independently, it is also suitable for use by male subjects with a low psychological predisposition to meet a doctor and/or who for logistical reasons do not have the opportunity,
- it guarantees full safety and comfort during all stages of the test,
- it is applicable to a large number of users, thanks to the fact that it is not a vehicle for infections or does not cause allergic reactions,
- does not require an clinic with specialized personnel and complex dedicated instrumentation and connected/managed by a computer,
- does not provide for detection by means of a fiber optic probe or by means of electromagnetic sensors, and is therefore simpler and cheaper to manufacture,
- allows you to implement screening as an alternative to DRE, with levels of standardization of the result that solve the limits linked to the subjectivity of DRE,
- detects the alterations/variations in consistency, volumetric/dimensional and/or morphological of the prostate gland; suitably, it allows to measure the consistency alterations (in particular in terms of elastic stiffness/compliance), and not only dimensional, of the tissues of said gland.

In particular, the solution according to the present invention is aimed at detecting the characteristics of the prostate and does not provide for any electrical neurostimulation, unlike instead of EP2322110 which measures neuronal reactivity, and in particular the reflex to an electrical stimulus, of the rectal and of those between it and the urethra and, for this purpose, the device is provided with means for electrical neurostimulation of the tissues.

Furthermore, in the solution of EP2322110, the pressure sensor is positioned inside the balloon and measures the macroscopic variation of the pressure inside the balloon itself following the deformation of said balloon deriving from the contact of the latter with the rectal walls which they react to an electrical impulse applied by the device itself. In particular, in the solution of EP2322110, direct contact of the pressure sensor with the organ tissue is not provided. On the contrary, in the solution according to the present invention, the expandable element acts as a transport element to move the pressure sensor and bring it into direct contact with the prostate.

## Claims

1. Device (1) for detecting the characteristics of the prostate, in particular in order to identify potentially pathological conditions of the prostate itself, including:
- an insertion element (12) that is intended to be inserted through the anus into a person' s rectum,
- a pressure sensor (20) which is mounted on an expandable element (18) and which is configured to detect the elasticity of the prostate tissues of said subject and/or at least a morphological and/or dimensional characteristic of the prostate of said subject,
- said expandable element (18) which is configured to assume a first condition, which is suitable for allowing said expandable element (18) to be housed - wholly or mostly - in the insertion element (12), and a second a condition that is such as to bring said pressure sensor (20), mounted on said expandable element (18), into contact/adherence with the prostate of said subject,
- an extractor means (30) which is associated with the insertion element (12) and which is intended to always remain, at least in part, external to the anus of said subject,
- an introducer element (2) which is configured to be inserted, at least partially, inside the anus of said subject for stabilization of the device with respect to the anus of said subject, said insertion element (12) being slidably movable with respect to said introducer element (2),
and **characterized in that** the introducer element (2) comprises a shaped body which is internally hollow, so as to enable the sliding crossing by the insertion element (12) inside the introducer element (2) during introduction of the insertion element (12) into the rectum without the insertion element (12) coming into contact with the internal walls of the anus, and which is configured both in terms of shape and size so as to be anatomically hooked and stabilized to the anus of said subject.

2. Device according to claim 1, **characterized in that** said insertion element (12) has a substantially finger-like conformation and comprises:
- an internal cavity (21) for housing said expandable element (18) in said first condition, and
- an opening (23) for the exit of said expandable element (18) from said internal cavity (21), to make it pass into said second condition.

3. Device according to one or more of the preceding claims, **characterized in that** it comprises:
- a first configuration, preferably corresponding to an inactive and/or disconnection configuration of said device, in which the introducer element (2) is in contact with or is near the end (14) of said insertion element (12) which is intended to be inserted inside the rectum of said subject,
- a second configuration, preferably corresponding to an insertion configuration of said device, in which the introducer element (2), intended to be positioned on the anus of said subject, is further spaced from the end (14) of said insertion element (12) which is intended to be inserted inside the rectum of said subject,
and by the fact that the passage from said first configuration to said second configuration, and vice versa, occurs by sliding the insertion element (12) with respect to said introducer (2).

4. Device according to claim 3,
**characterized in that** it comprises locking means (34) for stably locking the introducer (2) and the insertion element (12) in said first configuration and/or in said second configuration of said device (1), thus preventing sliding of the insertion element (12) with respect to the introducer (2).

5. Device according to one or more of the preceding claims, **characterized in that** said expandable element (18) comprises an inflatable bag (18') which is associated with means for its inflation/deflation and on which said pressure sensor (20).

6. Device according to one or more of the preceding claims, **characterized in that** said pressure sensor (20) comprises a laminar element which is sensitive to pressure and which is configured to change color according to the pressure exerted externally on it as a result of the its contact with the prostate tissues.

7. Device according to one or more of the preceding claims, **characterized in that** said extractor means (30) comprises a handle (26) which is angled with respect to said insertion element (12).

8. Device according to one or more of the preceding claims, **characterized in that** said extractor means (30) comprises a handle (26) which is articulated to said insertion element (12) by means of a hinge (29) which allows to adjust the angle (α) defined between said handle (26) and said insertion element (12).

9. A device according to claim 3, **characterized in that** it is configured so that said expandable element (18) passes into said second expanded condition only when the device is in said second configuration and/or **in that** the device is configured in such a way that its passage from the second to the first configuration takes place only when said expandable element (18) is located in said first retracted/contracted condition.

10. Device according to one or more of the preceding claims, **characterized in that** said introducer element (2) internally comprises a through axial cavity (10) within which the insertion element (12) slides.

11. Device according to one or more of the preceding claims, **characterized in that** the introducer element (2) comprises:
- a swollen upper part (4) configured to be fully inserted inside the anus of said subject,
- a flattened lower part (6), with a larger diameter than the swollen upper part (4), and configured to rest externally on the perineal area of said subject,
- an intermediate neck (8) for connection between said swollen upper part (4) and said flattened lower part (6) and configured to be positioned and retained by the anal sphincter of said subject.

12. Device according to one or more of the preceding claims, **characterized in that** the upper end (14) form of the insertion element (12) and the shape of the upper part (4) of the introducer (2) are such that, in first device inactive configuration (1), define a substantial continuity of the external profile.

13. Device according to one or more of the preceding claims, **characterized in that** said expandable element (18) is made, at least in part, in a shape memory materials.

14. Device according to one or more of the preceding claims, **characterized in that** said at least one pressure sensor (20) is applied on the external surface/area of the expandable element (18) which is intended to be brought near and/or in contact with the prostate of said subject.

15. Device according to one or more of the preceding claims, **characterized in that** said extractor means (30) can also be configured to cause the insertion element (12) to slide relative to the introducer element (2), to make it pass the device from said first configuration to said second configuration and/or vice versa.

## Patentansprüche

1. Vorrichtung (1) zur Erkennung von Eigenschaften der Prostata, insbesondere zur Erkennung potentiell pathologischer Zustände der Prostata selbst, umfassend:
- ein Einführelement (12), das dazu vorgesehen ist, durch den Anus in das Rektum einer Person eingeführt zu werden,
- einen Drucksensor (20), der auf einem ausdehnbaren Element (18 ) angebracht ist und der dazu ausgebildet ist, die Elastizität des Prostatagewebes des Subjekts und/oder zumindest eine morphologische und/oder dimensionale Eigenschaft der Prostata des Subjekts zu erfassen,
- wobei das erweiterbare Element (18) so konfiguriert ist, dass es einen ersten Zustand einnimmt, der dazu geeignet ist, dass das erweiterbare Element (18) ganz oder größtenteils im Einführelement (12) untergebracht werden kann, und einen zweiten Zustand, der dazu geeignet ist, den auf dem erweiterbaren Element (18) montierten Drucksensor (20) in Kontakt mit/an der Prostata des Patienten zu bringen,
- ein Extraktionsmittel (30), das mit dem Einführelement (12) verbunden ist und dazu bestimmt ist, immer zumindest teilweise außerhalb des Anus des Subjekts zu verbleiben,
- ein Einführelement (2), das so konfiguriert ist, dass es zumindest teilweise in den Anus des Subjekts eingeführt wird, um die Vorrichtung in Bezug auf den Anus des Subjekts zu stabilisieren, wobei das Einführelement (12) in Bezug auf das Einführelement (2) verschiebbar ist,
und **dadurch gekennzeichnet, dass** das Einführelement (2) einen geformten Körper umfasst, der innen hohl ist, um das gleitende Durchqueren des Einführelements (12) im Inneren des Einführelements (2) während der Einführung des Einführelements (12) in das Rektum zu ermöglichen, ohne dass das Einführelement (12) mit den Innenwänden des Anus in Berührung kommt, und der sowohl hinsichtlich der Form als auch der Größe so konfiguriert ist, dass er anatomisch am Anus des Subjekts eingehakt und dort stabilisiert wird.

2. Vorrichtung nach Anspruch 1, **dadurch gekennzeichnet, dass** das Einführelement (12) eine im Wesentlichen fingerartige Gestalt aufweist und umfasst:
- einen inneren Hohlraum (21) zur Aufnahme des ausdehnbaren Elements (18) in dem ersten Zustand, und
- eine Öffnung (23) für den Austritt des ausdehnbaren Elements (18) aus dem inneren Hohlraum (21), um es in den zweiten Zustand überzuführen.

3. Vorrichtung nach einem oder mehreren der vorhergehenden Ansprüche, **dadurch gekennzeichnet, dass** sie umfasst:
- eine erste Konfiguration, die vorzugsweise einer inaktiven und/oder Trennungskonfiguration der Vorrichtung entspricht, in der das Einführelement (2) mit dem Ende (14) des Einführelements (12) in Kontakt steht oder sich in dessen Nähe befindet, das in das Rektum des Patienten eingeführt werden soll,
- eine zweite Konfiguration, die vorzugsweise einer Einführkonfiguration der Vorrichtung entspricht, in der das Einführelement (2), das am Anus des Subjekts positioniert werden soll, weiter vom Ende (14) des Einführelements (12) entfernt ist, das in das Rektum des Subjekts eingeführt werden soll,
und durch die Tatsache, dass der Übergang von der ersten Konfiguration in die zweite Konfiguration und umgekehrt durch Verschieben des Einführelements (12) in Bezug auf den Einführer (2) erfolgt.

4. Vorrichtung nach Anspruch 3, **dadurch gekennzeichnet, dass** sie Verriegelungsmittel (34) zum stabilen Verriegeln des Einführers (2) und des Einführelements (12) in der ersten Konfiguration und/oder in der zweiten Konfiguration der Vorrichtung (1) umfasst, wodurch ein Gleiten des Einführelements (12) in Bezug auf den Einführer (2) verhindert wird.

5. Vorrichtung nach einem oder mehreren der vorhergehenden Ansprüche, **dadurch gekennzeichnet, dass** das ausdehnbare Element (18) einen aufblasbaren Beutel (18') umfasst, der mit Mitteln zu seinem Aufblasen/Entleeren verbunden ist und an dem sich der Drucksensor (20) befindet.

6. Vorrichtung nach einem oder mehreren der vorhergehenden Ansprüche, **dadurch gekennzeichnet, dass** der Drucksensor (20) ein druckempfindliches laminares Element umfasst, das so konfiguriert ist, dass es je nach dem von außen auf es ausgeübten Druck aufgrund seines Kontakts mit dem Prostatagewebe seine Farbe ändert.

7. Vorrichtung nach einem oder mehreren der vorhergehenden Ansprüche, **dadurch gekennzeichnet, dass** die Extraktionseinrichtung (30) einen Griff (26) aufweist, der in Bezug auf das Einführelement (12) abgewinkelt ist.

8. Vorrichtung nach einem oder mehreren der vorhergehenden Ansprüche, **dadurch gekennzeichnet, dass** die Extraktionseinrichtung (30) einen Griff (26) umfasst, der mittels eines Scharniers (29) an dem Einführelement (12) angelenkt ist, wodurch der zwischen dem Griff (26) und dem Einführelement (12) definierte Winkel ( α ) eingestellt werden kann.

9. Vorrichtung nach Anspruch 3, **dadurch gekennzeichnet, dass** sie so konfiguriert ist, dass das ausdehnbare Element (18) nur dann in den zweiten ausgedehnten Zustand übergeht, wenn sich die Vorrichtung in der zweiten Konfiguration befindet und/oder dass die Vorrichtung so konfiguriert ist, dass ihr Übergang von der zweiten in die erste Konfiguration nur dann stattfindet, wenn sich das ausdehnbare Element (18) in dem ersten eingefahrenen/kontrahierten Zustand befindet.

10. Vorrichtung nach einem oder mehreren der vorhergehenden Ansprüche, **dadurch gekennzeichnet, dass** das Einführelement (2) im Inneren einen durchgehenden axialen Hohlraum (10) aufweist, in dem das Einführelement (12) gleitet.

11. Vorrichtung nach einem oder mehreren der vorhergehenden Ansprüche, **dadurch gekennzeichnet, dass** das Einführelement (2) umfasst:
- ein geschwollenes Oberteil (4), das so konfiguriert ist, dass es vollständig in den Anus des Subjekts eingeführt werden kann,
- einen abgeflachten unteren Teil (6), der einen größeren Durchmesser als der geschwollene obere Teil (4) aufweist und so konfiguriert ist, dass er außen auf dem Dammbereich des Subjekts aufliegt,
- einen Zwischenhals (8) zur Verbindung zwischen dem geschwollenen oberen Teil (4) und dem abgeflachten unteren Teil (6), der so konfiguriert ist, dass er vom Analschließmuskel des Patienten positioniert und gehalten wird.

12. Vorrichtung nach einem oder mehreren der vorhergehenden Ansprüche, **dadurch gekennzeichnet, dass** die Form des oberen Endes (14) des Einführelements (12) und die Form des oberen Teils (4) des Einführinstruments (2) derart sind, dass sie in der inaktiven Konfiguration der ersten Vorrichtung (1) eine wesentliche Kontinuität des Außenprofils definieren.

13. Vorrichtung nach einem oder mehreren der vorhergehenden Ansprüche, **dadurch gekennzeichnet, dass** das ausdehnbare Element (18) zumindest teilweise aus einem Formgedächtnismaterial besteht.

14. Vorrichtung nach einem oder mehreren der vorhergehenden Ansprüche, **dadurch gekennzeichnet, dass** der mindestens eine Drucksensor (20) auf der äußeren Oberfläche/Fläche des erweiterbaren Elements (18) angebracht ist, die dazu bestimmt ist, in die Nähe der Prostata des Subjekts gebracht zu werden und/oder mit dieser in Kontakt zu kommen.

15. Vorrichtung nach einem oder mehreren der vorhergehenden Ansprüche, **dadurch gekennzeichnet, dass** die Extraktionseinrichtung (30) auch so konfiguriert sein kann, dass sie das Einführelement (12) relativ zum Einführelement (2) gleiten lässt, um die Vorrichtung aus der ersten Konfiguration in die zweite Konfiguration und/oder umgekehrt zu überführen.

## Revendications

1. Dispositif (1) pour détecter les caractéristiques de la prostate, notamment afin d'identifier des états potentiellement pathologiques de la prostate elle-même, parmi lesquels :
- un élément d'insertion (12) destiné à être inséré par l'anus dans le rectum d'une personne,
- un capteur de pression (20) qui est monté sur un élément extensible (18) et qui est configuré pour détecter l'élasticité des tissus prostatiques dudit sujet et/ou au moins une caractéristique morphologique et/ou dimensionnelle de la prostate dudit sujet,
- ledit élément extensible (18) qui est configuré pour assumer une première condition, qui est appropriée pour permettre audit élément extensible (18) d'être logé - entièrement ou principalement - dans l'élément d'insertion (12), et une seconde une condition qui est telle afin d'amener ledit capteur de pression (20), monté sur ledit élément extensible (18), en contact/adhérence avec la prostate dudit sujet,
- un moyen d'extraction (30) qui est associé à l'élément d'insertion (12) et qui est destiné à rester toujours, au moins en partie, extérieur à l'anus dudit sujet,
- un élément introducteur (2) qui est configuré pour être inséré, au moins partiellement, à l'intérieur de l'anus dudit sujet pour la stabilisation du dispositif par rapport à l'anus dudit sujet, ledit élément d'insertion (12) étant mobile de manière coulissante par rapport audit élément introducteur (2),
et **caractérisé en ce que** l'élément introducteur (2) comprend un corps conformé intérieurement creux, de manière à permettre le passage coulissant de l'élément introducteur (12) à l'intérieur de l'élément introducteur (2) lors de l'introduction de l'élément introducteur (12) dans le rectum sans que l'élément d'insertion (12) n'entre en contact avec les parois internes de l'anus, et qui est configuré tant en forme qu'en taille de manière à être anatomiquement accroché et stabilisé à l'anus dudit sujet.

2. Dispositif selon la revendication 1, **caractérisé en ce que** ledit élément d'insertion (12) a une conformation sensiblement en forme de doigt et comprend :
- une cavité interne (21) pour loger ledit élément extensible (18) dans ledit premier état, et
- une ouverture (23) pour la sortie dudit élément expansible (18) de ladite cavité interne (21), pour le faire passer dans ledit deuxième état.

3. Dispositif selon une ou plusieurs des revendications précédentes, **caractérisé en ce qu'**il comprend :
- une première configuration, correspondant de préférence à une configuration d'inactivité et/ou de déconnexion dudit dispositif, dans laquelle l'élément introducteur (2) est en contact ou à proximité de l'extrémité (14) dudit élément d'insertion (12) qui est destinée à être inséré à l'intérieur du rectum dudit sujet,
- une deuxième configuration, correspondant de préférence à une configuration d'insertion dudit dispositif, dans laquelle l'élément introducteur (2), destiné à être positionné sur l'anus dudit sujet, est en outre espacé de l'extrémité (14) dudit élément d'insertion (12) qui est destiné à être inséré à l'intérieur du rectum dudit sujet,
et par le fait que le passage de ladite première configuration à ladite deuxième configuration, et vice versa, s'effectue par coulissement de l'élément d'insertion (12) par rapport audit introducteur (2).

4. Dispositif selon la revendication 3, **caractérisé en ce qu'**il comprend des moyens de verrouillage (34) pour verrouiller de manière stable l'introducteur (2) et l'élément d'insertion (12) dans ladite première configuration et/ou dans ladite deuxième configuration dudit dispositif (1), empêchant ainsi le glissement de l'élément d'insertion (12) par rapport à l'introducteur (2).

5. Dispositif selon une ou plusieurs des revendications précédentes, **caractérisé en ce que** ledit élément expansible (18) comprend un sac gonflable (18') qui est associé à des moyens pour son gonflage/dégonflage et sur lequel repose ledit capteur de pression (20).

6. Dispositif selon une ou plusieurs des revendications précédentes, **caractérisé en ce que** ledit capteur de pression (20) comprend un élément laminaire sensible à la pression et qui est configuré pour changer de couleur en fonction de la pression exercée extérieurement sur lui suite à le son contact avec les tissus de la prostate.

7. Dispositif selon une ou plusieurs des revendications précédentes, **caractérisé en ce que** lesdits moyens d'extraction (30) comprennent une poignée (26) qui est inclinée par rapport audit élément d'insertion (12).

8. Dispositif selon une ou plusieurs des revendications précédentes, **caractérisé en ce que** ledit moyen d'extraction (30) comprend une poignée (26) qui est articulée audit élément d'insertion (12) au moyen d'une charnière (29) qui permet d'ajuster l'angle ( α ) défini entre ladite poignée (26) et ledit élément d'insertion (12).

9. Dispositif selon la revendication 3, **caractérisé en ce qu'**il est configuré pour que ledit élément expansible (18) passe dans ledit deuxième état déployé uniquement lorsque le dispositif est dans ladite deuxième configuration et/ou **en ce que** le dispositif est configuré dans un tel de sorte que son passage de la seconde à la première configuration s'effectue uniquement lorsque ledit élément extensible (18) est situé dans ledit premier état rétracté/contracté.

10. Dispositif selon une ou plusieurs des revendications précédentes, **caractérisé en ce que** ledit élément introducteur (2) comprend intérieurement une cavité axiale traversante (10) à l'intérieur de laquelle coulisse l'élément d'insertion (12).

11. Dispositif selon l'une ou plusieurs des revendications précédentes, **caractérisé en ce que** l'élément introducteur (2) comprend :
- une partie supérieure gonflée (4) configurée pour être entièrement insérée à l'intérieur de l'anus dudit sujet,
- une partie inférieure aplatie (6), de plus grand diamètre que la partie supérieure renflée (4), et configurée pour reposer extérieurement sur la zone périnéale dudit sujet,
- un col intermédiaire (8) de liaison entre ladite partie supérieure gonflée (4) et ladite partie inférieure aplatie (6) et configuré pour être positionné et retenu par le sphincter anal dudit sujet.

12. Dispositif selon une ou plusieurs des revendications précédentes, **caractérisé en ce que** la forme de l'extrémité supérieure (14) de l'élément d'insertion (12) et la forme de la partie supérieure (4) de l'introducteur (2) sont telles que, en premier configuration inactive du dispositif (1), définissent une continuité substantielle du profil extérieur.

13. Dispositif selon une ou plusieurs des revendications précédentes, **caractérisé en ce que** ledit élément expansible (18) est réalisé, au moins en partie, dans un matériaux à mémoire de forme .

14. Dispositif selon une ou plusieurs des revendications précédentes, **caractérisé en ce que** ledit au moins un capteur de pression (20) est appliqué sur la surface/zone externe de l'élément expansible (18) qui est destinée à être rapprochée et/ou en contact avec la prostate dudit sujet.

15. Dispositif selon une ou plusieurs des revendications précédentes, **caractérisé en ce que** lesdits moyens d'extraction (30) peuvent également être configurés pour faire coulisser l'élément d'insertion (12) par rapport à l'élément d'introduction (2), pour lui faire passer le dispositif de ladite première configuration à ladite deuxième configuration et/ou vice versa.
